# EUROPÄISCHE PATENTANMELDUNG

(11) **EP 0 962 528 A2**
(43) Veröffentlichungstag der Anmeldung: **08.12.1999**
(21) Anmeldenummer: 99107852.8
(22) Anmeldetag: 21.04.1999
(51) Int. Cl.: C12N 15/12, C07K 14/705, C07K 16/28, G01N 33/50, C12Q 1/68, C12N 5/10, A01K 67/033, C12N 15/85

(54) **Nukleisäuren, die für Acetylcholinrezeptor-Untereinheiten von Insekten kodieren**

(30) Priorität: 04.05.1998 DE 19819829
(71) Anmelder: Bayer Aktiengesellschaft, 51368 Leverkusen (DE)
(72) Erfinder: Adamczewski, Martin Dr., 51067 Köln (DE); Oellers, Nadja Dr., 50670 Köln (DE); Schulte, Thomas Dr., 51061 Köln (DE)

(57) **Zusammenfassung**

Die Erfindung betrifft Nukleinsäuren, die für Acetylcholinrezeptor-Untereinheiten von Insekten kodieren, die entsprechenden Polypeptide, sowie Verfahren zum Auffinden neuer Wirkstoffe für den Pflanzenschutz.

SEQUENZPROTOKOLL

<110> Bayer Aktiengesellschaft

<120> Nukleinsäuren, die für
      Acetylcholinrezeptor-Untereinheiten von Insekten
      kodieren

<130> Le A 33 020-EP

<140> 99107852.8
<141> 1999-04-21

<150> DE 198 19 829.9
<151> 1998-05-04

<160> 6

<170> PatentIn Ver. 2.1

<210> 1
<211> 2886
<212> DNA
<213> Drosophila melanogaster

<220>
<221> CDS
<222> (372)..(2681)

<400> 1
ggcacgagaa aaagttgtgg tataaacttt tattgtagga aaacgcataa aaataataga 60

aaaacgctct tcgggttgta aagaaaataa gaagacaaaa gaaagacatg aaaacgttgc 120

aaacaataaa gcatatactt gccatattga tataaaggga aatcgtgaaa aggcggtgaa 180

aatttcgtaa gattagttgg tattaagggc agcccatgca cacagctaaa aagggaacta 240

aaaaaacccc gcacagaaca atgaaagctg cagcagctgg ataaggccga caaaaccgaa 300

aattatatta ttgtaatcta gtagagagca gacaacatat ccgctggcaa caaccaacac 360

cgaaagagac t atg aaa aat gca caa ctg aaa ctg act gaa gtt gac gat 410
             Met Lys Asn Ala Gln Leu Lys Leu Thr Glu Val Asp Asp
               1               5                  10

gat gag ctg tgg ctg gca gta aga tta gcg cac tgc agc agc aac ttt 458
Asp Glu Leu Trp Leu Ala Val Arg Leu Ala His Cys Ser Ser Asn Phe

    15                  20                  25

agc agc agt agc agc aca aga acc acc agc agc aac cag agg cac aac 506
Ser Ser Ser Ser Ser Thr Arg Thr Thr Ser Ser Asn Gln Arg His Asn
30                  35                  40                  45

cag caa ctc aca aca ctg caa cca agg agc tta agt aca aaa cac cac 554
Gln Gln Leu Thr Thr Leu Gln Pro Arg Ser Leu Ser Thr Lys His His
                50                  55                  60

agc aac att gca agc gag cag cac aat agc cag caa cag gag cca gca 602
Ser Asn Ile Ala Ser Glu Gln His Asn Ser Gln Gln Gln Glu Pro Ala
            65                  70                  75

tcg aag gac gag gat gta gcc aac cac ggt aga agc aat gac cag cag 650
Ser Lys Asp Glu Asp Val Ala Asn His Gly Arg Ser Asn Asp Gln Gln
        80                  85                  90

acg cat ctg caa cag cta gac agc agc aac atg ttg tcg cca aag aca 698
Thr His Leu Gln Gln Leu Asp Ser Ser Asn Met Leu Ser Pro Lys Thr
    95                  100                 105

gcc gca gca gca act gct gcc ggc gat gaa gca aca acc caa caa cca 746
Ala Ala Ala Ala Thr Ala Ala Gly Asp Glu Ala Thr Thr Gln Gln Pro
110                 115                 120                 125

aca aac ata aga ctg tgt gca cgc aag cga caa cga ttg cgt cgc cga 794
Thr Asn Ile Arg Leu Cys Ala Arg Lys Arg Gln Arg Leu Arg Arg Arg
                130                 135                 140

cga aaa aga aaa cca gca acc cca aac gaa aca gat atc aag aaa caa 842
Arg Lys Arg Lys Pro Ala Thr Pro Asn Glu Thr Asp Ile Lys Lys Gln
            145                 150                 155

cag caa ctt agc atg cct ccc ttc aaa acg cgc aaa tcc acg gac acc 890
Gln Gln Leu Ser Met Pro Pro Phe Lys Thr Arg Lys Ser Thr Asp Thr
        160                 165                 170

tac agc aca cca gca gca aca acc agc tgt ccg aca gcc acc tac atg 938
Tyr Ser Thr Pro Ala Ala Thr Thr Ser Cys Pro Thr Ala Thr Tyr Met
    175                 180                 185

caa tgt cga gcc agc gac aat gag ttc agt att ccg ata tcg aga cat 986
Gln Cys Arg Ala Ser Asp Asn Glu Phe Ser Ile Pro Ile Ser Arg His
190                 195                 200                 205

gat aga gta tcc acg gcc aca ttc gcc tgg gtg ttg cat gtg ctg cag 1034
Asp Arg Val Ser Thr Ala Thr Phe Ala Trp Val Leu His Val Leu Gln

                210                 215                 220

gtg ctg ctc gtg tcg ctg caa cag tgg caa ctt cac gtg caa cag cga 1082
Val Leu Leu Val Ser Leu Gln Gln Trp Gln Leu His Val Gln Gln Arg
            225                 230                 235

tcg gtg cta ctg ttc aga agg atc gca gcg agc acc atc gcc ttc att 1130
Ser Val Leu Leu Phe Arg Arg Ile Ala Ala Ser Thr Ile Ala Phe Ile
        240                 245                 250

tcc tat tta ggc agc ttt gca gcg caa ctg aaa aat agc agc agc agc 1178
Ser Tyr Leu Gly Ser Phe Ala Ala Gln Leu Lys Asn Ser Ser Ser Ser
    255                 260                 265

agt agc agc agc aac agc agc aac aac agc agc acg caa ata tta aac 1226
Ser Ser Ser Ser Asn Ser Ser Asn Asn Ser Ser Thr Gln Ile Leu Asn
270                 275                 280                 285

gga ctt aat aaa cac tca tgg ata ttt tta ttg ata tat ttg aat tta 1274
Gly Leu Asn Lys His Ser Trp Ile Phe Leu Leu Ile Tyr Leu Asn Leu
                290                 295                 300

tct gct aaa gtt tgc cta gca gga tat cat gaa aag aga ctg tta cac 1322
Ser Ala Lys Val Cys Leu Ala Gly Tyr His Glu Lys Arg Leu Leu His
            305                 310                 315

gat ctt ttg gat cct tat aat aca cta gaa cgt ccc gtt ctc aat gaa 1370
Asp Leu Leu Asp Pro Tyr Asn Thr Leu Glu Arg Pro Val Leu Asn Glu
        320                 325                 330

tcg gac ccg tta caa tta agc ttt ggt tta act tta atg caa att atc 1418
Ser Asp Pro Leu Gln Leu Ser Phe Gly Leu Thr Leu Met Gln Ile Ile
    335                 340                 345

gat gtg gac gag aaa aat caa ttg cta gtc act aat gtg tgg tta aaa 1466
Asp Val Asp Glu Lys Asn Gln Leu Leu Val Thr Asn Val Trp Leu Lys
350                 355                 360                 365

ctg gag tgg aac gac atg aat ctc cgc tgg aac acc tcc gac tat ggc 1514
Leu Glu Trp Asn Asp Met Asn Leu Arg Trp Asn Thr Ser Asp Tyr Gly
                370                 375                 380

gga gtt aag gat ctg cga ata ccg ccg cat cgc atc tgg aag ccg gac 1562
Gly Val Lys Asp Leu Arg Ile Pro Pro His Arg Ile Trp Lys Pro Asp
            385                 390                 395

gtg ctg atg tac aac agt gcg gat gag gga ttt gac ggc acc tac cag 1610
Val Leu Met Tyr Asn Ser Ala Asp Glu Gly Phe Asp Gly Thr Tyr Gln

        400                 405                 410

acg aac gtg gtg gtg cgg aac aac ggc tcg tgt cta tac gtt ccg ccg 1658
Thr Asn Val Val Val Arg Asn Asn Gly Ser Cys Leu Tyr Val Pro Pro
    415                 420                 425

ggg atc ttc aag tcg acg tgc aag atc gac atc acg tgg ttc ccc ttc 1706
Gly Ile Phe Lys Ser Thr Cys Lys Ile Asp Ile Thr Trp Phe Pro Phe
430                 435                 440                 445

gat gac cag cgg tgc gag atg aag ttc ggc agt tgg acc tac gac gga 1754
Asp Asp Gln Arg Cys Glu Met Lys Phe Gly Ser Trp Thr Tyr Asp Gly
                450                 455                 460

ttc cag ctg gat tta caa tta caa gat gaa act ggc ggt gat atc agc 1802
Phe Gln Leu Asp Leu Gln Leu Gln Asp Glu Thr Gly Gly Asp Ile Ser
            465                 470                 475

agt tac gtg ctc aac ggc gag tgg gaa cta ctg ggt gtg ccc ggc aaa 1850
Ser Tyr Val Leu Asn Gly Glu Trp Glu Leu Leu Gly Val Pro Gly Lys
        480                 485                 490

cgt aac gag atc tat tac aac tgc tgc ccg gaa ccc tat ata gac atc 1898
Arg Asn Glu Ile Tyr Tyr Asn Cys Cys Pro Glu Pro Tyr Ile Asp Ile
    495                 500                 505

acc ttc gcc atc atc atc cgc cga cga aca ctg tac tat ttc ttc aac 1946
Thr Phe Ala Ile Ile Ile Arg Arg Arg Thr Leu Tyr Tyr Phe Phe Asn
510                 515                 520                 525

ctg atc ata cct tgt gta ctg att gcc tcc atg gcc ttg ctc gga ttc 1994
Leu Ile Ile Pro Cys Val Leu Ile Ala Ser Met Ala Leu Leu Gly Phe
                530                 535                 540

acc ctg ccg cca gat tcg ggt gaa aaa tta tcg ctg ggt gtt acc atc 2042
Thr Leu Pro Pro Asp Ser Gly Glu Lys Leu Ser Leu Gly Val Thr Ile
            545                 550                 555

ttg ctc tcg ctg acc gtg ttt ctg aat atg gtt gcc gag aca atg ccg 2090
Leu Leu Ser Leu Thr Val Phe Leu Asn Met Val Ala Glu Thr Met Pro
        560                 565                 570

gct act tcc gat gcg gtg cca ttg tgg ata cgc atc gtg ttt ttg tgc 2138
Ala Thr Ser Asp Ala Val Pro Leu Trp Ile Arg Ile Val Phe Leu Cys
    575                 580                 585

tgg ctg cca tgg ata ttg cga atg agt cgc cca gga cga ccg ctg atc 2186
Trp Leu Pro Trp Ile Leu Arg Met Ser Arg Pro Gly Arg Pro Leu Ile

590                 595                 600                 605

cta gag ttc ccg acc acg ccc tgt tcg gac aca tcc tcc gag cgg aag    2234
Leu Glu Phe Pro Thr Thr Pro Cys Ser Asp Thr Ser Ser Glu Arg Lys
                610                 615                 620

cac cag ata ctc tcc gac gtt gag ctg aaa gag cgc tcg tcg aaa tcg    2282
His Gln Ile Leu Ser Asp Val Glu Leu Lys Glu Arg Ser Ser Lys Ser
            625                 630                 635

ctg ctg gcc aac gta cta gac atc gat gat gac ttc cgg cac aat tgt    2330
Leu Leu Ala Asn Val Leu Asp Ile Asp Asp Asp Phe Arg His Asn Cys
        640                 645                 650

cgc ccc atg acg ccc ggc gga aca ctg cca cac aac ccg gct ttc tat    2378
Arg Pro Met Thr Pro Gly Gly Thr Leu Pro His Asn Pro Ala Phe Tyr
    655                 660                 665

cgc acg gtt tat gga caa ggc gac gat ggc agc att ggg cca att ggc    2426
Arg Thr Val Tyr Gly Gln Gly Asp Asp Gly Ser Ile Gly Pro Ile Gly
670                 675                 680                 685

agc acc cga atg ccg gat gcg gtc acc cat cat acg tgc atc aaa tca    2474
Ser Thr Arg Met Pro Asp Ala Val Thr His His Thr Cys Ile Lys Ser
                690                 695                 700

tca act gaa tat gaa tta ggt tta atc tta aag gaa att cgc ttt ata    2522
Ser Thr Glu Tyr Glu Leu Gly Leu Ile Leu Lys Glu Ile Arg Phe Ile
            705                 710                 715

act gat cag cta cgt aaa gat gac gag tgc aat gac att gcc aat gat    2570
Thr Asp Gln Leu Arg Lys Asp Asp Glu Cys Asn Asp Ile Ala Asn Asp
        720                 725                 730

tgg aaa ttt gca gct atg gtc gtt gac aga ctg tgc ctt atc ata ttc    2618
Trp Lys Phe Ala Ala Met Val Val Asp Arg Leu Cys Leu Ile Ile Phe
    735                 740                 745

aca atg ttc gca ata tta gcc aca ata gct gta cta cta tcg gca cca    2666
Thr Met Phe Ala Ile Leu Ala Thr Ile Ala Val Leu Leu Ser Ala Pro
750                 755                 760                 765

cat att att gtc tcg tagccatatg ggcgaggtgg ttattgttat tggttttatt    2721
His Ile Ile Val Ser
                770

ataaaatcaa tttgttaatt attaaattaa taacgaaact ctttaagtaa attaaaacta 2781

aaaagacact aaaaaagcac aaaaaaatag gaaaatacat gataaaaccc atgaactaaa 2841

taatacatcc aagaaaaacc aaaacaaaaa aaaaaaaaaa aaaaa 2886

<210> 2
<211> 770
<212> PRT
<213> Drosophila melanogaster

<400> 2
Met Lys Asn Ala Gln Leu Lys Leu Thr Glu Val Asp Asp Asp Glu Leu
1               5                   10                  15

Trp Leu Ala Val Arg Leu Ala His Cys Ser Ser Asn Phe Ser Ser Ser
            20                  25                  30

Ser Ser Thr Arg Thr Thr Ser Ser Asn Gln Arg His Asn Gln Gln Leu
        35                  40                  45

Thr Thr Leu Gln Pro Arg Ser Leu Ser Thr Lys His His Ser Asn Ile
    50                  55                  60

Ala Ser Glu Gln His Asn Ser Gln Gln Gln Glu Pro Ala Ser Lys Asp
65                  70                  75                  80

Glu Asp Val Ala Asn His Gly Arg Ser Asn Asp Gln Gln Thr His Leu
                85                  90                  95

Gln Gln Leu Asp Ser Ser Asn Met Leu Ser Pro Lys Thr Ala Ala Ala
            100                 105                 110

Ala Thr Ala Ala Gly Asp Glu Ala Thr Thr Gln Gln Pro Thr Asn Ile
        115                 120                 125

Arg Leu Cys Ala Arg Lys Arg Gln Arg Leu Arg Arg Arg Arg Lys Arg
    130                 135                 140

Lys Pro Ala Thr Pro Asn Glu Thr Asp Ile Lys Lys Gln Gln Gln Leu
145                 150                 155                 160

Ser Met Pro Pro Phe Lys Thr Arg Lys Ser Thr Asp Thr Tyr Ser Thr
                165                 170                 175

Pro Ala Ala Thr Thr Ser Cys Pro Thr Ala Thr Tyr Met Gln Cys Arg
            180                 185                 190

Ala Ser Asp Asn Glu Phe Ser Ile Pro Ile Ser Arg His Asp Arg Val

        195                 200                 205

Ser Thr Ala Thr Phe Ala Trp Val Leu His Val Leu Gln Val Leu Leu
    210                 215                 220

Val Ser Leu Gln Gln Trp Gln Leu His Val Gln Gln Arg Ser Val Leu
225                 230                 235                 240

Leu Phe Arg Arg Ile Ala Ala Ser Thr Ile Ala Phe Ile Ser Tyr Leu
                245                 250                 255

Gly Ser Phe Ala Ala Gln Leu Lys Asn Ser Ser Ser Ser Ser Ser Ser
            260                 265                 270

Ser Asn Ser Ser Asn Asn Ser Ser Thr Gln Ile Leu Asn Gly Leu Asn
        275                 280                 285

Lys His Ser Trp Ile Phe Leu Leu Ile Tyr Leu Asn Leu Ser Ala Lys
    290                 295                 300

Val Cys Leu Ala Gly Tyr His Glu Lys Arg Leu Leu His Asp Leu Leu
305                 310                 315                 320

Asp Pro Tyr Asn Thr Leu Glu Arg Pro Val Leu Asn Glu Ser Asp Pro
                325                 330                 335

Leu Gln Leu Ser Phe Gly Leu Thr Leu Met Gln Ile Ile Asp Val Asp
            340                 345                 350

Glu Lys Asn Gln Leu Leu Val Thr Asn Val Trp Leu Lys Leu Glu Trp
        355                 360                 365

Asn Asp Met Asn Leu Arg Trp Asn Thr Ser Asp Tyr Gly Gly Val Lys
    370                 375                 380

Asp Leu Arg Ile Pro Pro His Arg Ile Trp Lys Pro Asp Val Leu Met
385                 390                 395                 400

Tyr Asn Ser Ala Asp Glu Gly Phe Asp Gly Thr Tyr Gln Thr Asn Val
                405                 410                 415

Val Val Arg Asn Asn Gly Ser Cys Leu Tyr Val Pro Pro Gly Ile Phe
            420                 425                 430

Lys Ser Thr Cys Lys Ile Asp Ile Thr Trp Phe Pro Phe Asp Asp Gln
        435                 440                 445

Arg Cys Glu Met Lys Phe Gly Ser Trp Thr Tyr Asp Gly Phe Gln Leu

450                 455                 460

Asp Leu Gln Leu Gln Asp Glu Thr Gly Gly Asp Ile Ser Ser Tyr Val
465                 470                 475                 480

Leu Asn Gly Glu Trp Glu Leu Leu Gly Val Pro Gly Lys Arg Asn Glu
                485                 490                 495

Ile Tyr Tyr Asn Cys Cys Pro Glu Pro Tyr Ile Asp Ile Thr Phe Ala
            500                 505                 510

Ile Ile Ile Arg Arg Arg Thr Leu Tyr Tyr Phe Phe Asn Leu Ile Ile
        515                 520                 525

Pro Cys Val Leu Ile Ala Ser Met Ala Leu Leu Gly Phe Thr Leu Pro
    530                 535                 540

Pro Asp Ser Gly Glu Lys Leu Ser Leu Gly Val Thr Ile Leu Leu Ser
545                 550                 555                 560

Leu Thr Val Phe Leu Asn Met Val Ala Glu Thr Met Pro Ala Thr Ser
                565                 570                 575

Asp Ala Val Pro Leu Trp Ile Arg Ile Val Phe Leu Cys Trp Leu Pro
            580                 585                 590

Trp Ile Leu Arg Met Ser Arg Pro Gly Arg Pro Leu Ile Leu Glu Phe
    .   595                 600                 605

Pro Thr Thr Pro Cys Ser Asp Thr Ser Ser Glu Arg Lys His Gln Ile
    610                 615                 620

Leu Ser Asp Val Glu Leu Lys Glu Arg Ser Ser Lys Ser Leu Leu Ala
625                 630                 635                 640

Asn Val Leu Asp Ile Asp Asp Asp Phe Arg His Asn Cys Arg Pro Met
                645                 650                 655

Thr Pro Gly Gly Thr Leu Pro His Asn Pro Ala Phe Tyr Arg Thr Val
            660                 665                 670

Tyr Gly Gln Gly Asp Asp Gly Ser Ile Gly Pro Ile Gly Ser Thr Arg
        675                 680                 685

Met Pro Asp Ala Val Thr His His Thr Cys Ile Lys Ser Ser Thr Glu
    690                 695                 700

Tyr Glu Leu Gly Leu Ile Leu Lys Glu Ile Arg Phe Ile Thr Asp Gln

705                 710                 715                 720

Leu Arg Lys Asp Asp Glu Cys Asn Asp Ile Ala Asn Asp Trp Lys Phe
                725                 730                 735

Ala Ala Met Val Val Asp Arg Leu Cys Leu Ile Ile Phe Thr Met Phe
            740                 745                 750

Ala Ile Leu Ala Thr Ile Ala Val Leu Leu Ser Ala Pro His Ile Ile
        755                 760                 765

Val Ser
    770

<210> 3
<211> 3700
<212> DNA
<213> Heliothis virescens

<220>
<221> CDS
<222> (335)..(1822)

<400> 3
ggcacgagcc gctgccccac ggtcggccgc actccgctga acaacaatgc tcaaaaacac 60

gccgtgactc cacacacatc ccctcggcgc agtaggcgat gtttgaggat cggacggcac 120

gcgtggccgt cggcgagcgg tcgtgaacaa gttgcataca tatgaaaacc gtaaaaagat 180

tgaattttaa gccgatcgtg ttcgatagat cctaatagag aagcgggagt gcggcgtttg 240

gtaggcgggg gtcgagtcgc gcggtcgggg gaaatggcgc ggcgcggggc ggcggcggcg 300

gcggcgcgcg gcgcggcggc gtcgcggcgc tgac atg ggc ggg cgg gcg cgc cgc 355
                                      Met Gly Gly Arg Ala Arg Arg
                                      1               5

tcg cac ttg gcg gcg ccc gcg ggc ctg ctg ctg ctg ctg tgc ctg ctc    403
Ser His Leu Ala Ala Pro Ala Gly Leu Leu Leu Leu Leu Cys Leu Leu
        10                  15                  20

tgg ccg agg ggg gca cgc tgc ggg tac cac gag aag cgg cta ctg cac    451
Trp Pro Arg Gly Ala Arg Cys Gly Tyr His Glu Lys Arg Leu Leu His
    25                  30                  35

cac cta ttg gac cac tac aac gta ctg gag agg ccc gtc gtc aac gag 499
His Leu Leu Asp His Tyr Asn Val Leu Glu Arg Pro Val Val Asn Glu
40                  45                  50                  55

agc gac ccg ctg cag ctc tcc ttc ggc ctc acg ctc atg cag atc atc 547
Ser Asp Pro Leu Gln Leu Ser Phe Gly Leu Thr Leu Met Gln Ile Ile
                60                  65                  70

gac gtg gac gag aag aac cag ctt tta ata aca aac atc tgg cta aaa 595
Asp Val Asp Glu Lys Asn Gln Leu Leu Ile Thr Asn Ile Trp Leu Lys
            75                  80                  85

cta gag tgg aat gat atg aac ttg agg tgg aac act tca gat ttc ggc 643
Leu Glu Trp Asn Asp Met Asn Leu Arg Trp Asn Thr Ser Asp Phe Gly
        90                  95                  100

ggg gtc aaa gat tta aga gtg cca ccc cac aga cta tgg aaa cca gac 691
Gly Val Lys Asp Leu Arg Val Pro Pro His Arg Leu Trp Lys Pro Asp
    105                 110                 115

gtc ctt atg tac aac agc gcg gac gaa ggg ttc gac agc acg tat cca 739
Val Leu Met Tyr Asn Ser Ala Asp Glu Gly Phe Asp Ser Thr Tyr Pro
120                 125                 130                 135

acg aac gtg gtg gtg cgg aac aac ggc tcg tgt ctg tac gtg ccg ccc 787
Thr Asn Val Val Val Arg Asn Asn Gly Ser Cys Leu Tyr Val Pro Pro
                140                 145                 150

ggc atc ttc aag agc acc tgc aag atc gac atc acc tgg ttc ccc ttc 835
Gly Ile Phe Lys Ser Thr Cys Lys Ile Asp Ile Thr Trp Phe Pro Phe
            155                 160                 165

gac gac caa cga tgc gag atg aag ttt ggc agc tgg act tat gat ggt 883
Asp Asp Gln Arg Cys Glu Met Lys Phe Gly Ser Trp Thr Tyr Asp Gly
        170                 175                 180

tat cag ttg gat cta caa cta cag gat gaa ggg ggc gga gat ata agc 931
Tyr Gln Leu Asp Leu Gln Leu Gln Asp Glu Gly Gly Gly Asp Ile Ser
    185                 190                 195

agt ttt gtc acg aat ggc gaa tgg gag tta ata gga gtc ccc ggc aag 979
Ser Phe Val Thr Asn Gly Glu Trp Glu Leu Ile Gly Val Pro Gly Lys
200                 205                 210                 215

cgc aac gag atc tac tac aac tgt tgt ccg gag cca tac atc gac atc 1027
Arg Asn Glu Ile Tyr Tyr Asn Cys Cys Pro Glu Pro Tyr Ile Asp Ile
                220                 225                 230

acg ttt gcg gtg gtg atc cgg agg aaa acg ctc tac tac ttc ttc aat 1075
Thr Phe Ala Val Val Ile Arg Arg Lys Thr Leu Tyr Tyr Phe Phe Asn
            235                 240                 245

ctg atc gtg ccc tgc gtg ctc atc gcc tcc atg gct cta ttg ggg ttc 1123
Leu Ile Val Pro Cys Val Leu Ile Ala Ser Met Ala Leu Leu Gly Phe
        250                 255                 260

acc ttg cct cca gac tcc gga gaa aag ttg tct tta ggt gtg acg ata 1171
Thr Leu Pro Pro Asp Ser Gly Glu Lys Leu Ser Leu Gly Val Thr Ile
    265                 270                 275

tta ctg tcg ttg acg gtg ttc ctc aac atg gtg gcg gag acg atg cca 1219
Leu Leu Ser Leu Thr Val Phe Leu Asn Met Val Ala Glu Thr Met Pro
280                 285                 290                 295

gcg acg tcg gac gcc gtg ccc ttg ctc ggc acc tac ttc aac tgc atc 1267
Ala Thr Ser Asp Ala Val Pro Leu Leu Gly Thr Tyr Phe Asn Cys Ile
                300                 305                 310

atg ttc atg gtg gct tcc tcc gtc gtc tcc acc ata ctg atc ctc aac 1315
Met Phe Met Val Ala Ser Ser Val Val Ser Thr Ile Leu Ile Leu Asn
            315                 320                 325

tac cac cac cgg cac gca gac act cac gaa atg agt gat tgg att cgt 1363
Tyr His His Arg His Ala Asp Thr His Glu Met Ser Asp Trp Ile Arg
        330                 335                 340

tgc gtg ttc ctt tat tgg ctg ccg tgg gtg ctg cgc atg tca cgg ccc 1411
Cys Val Phe Leu Tyr Trp Leu Pro Trp Val Leu Arg Met Ser Arg Pro
    345                 350                 355

ggc tcg gcg acg acg ccg ccg ccg gcg cgc gta cct ccg ccg ccg gac 1459
Gly Ser Ala Thr Thr Pro Pro Pro Ala Arg Val Pro Pro Pro Pro Asp
360                 365                 370                 375

ctg gag ctg cgc gag cgc tcc tcc aag tcg ctc cta gcg aac gtg ctc 1507
Leu Glu Leu Arg Glu Arg Ser Ser Lys Ser Leu Leu Ala Asn Val Leu
                380                 385                 390

gac atc gat gac gac ttc cgc cac ccg caa gcg cag cag ccg caa tgc 1555
Asp Ile Asp Asp Asp Phe Arg His Pro Gln Ala Gln Gln Pro Gln Cys
            395                 400                 405

tgc cga tac tac agg ggg ggt gag gag aat ggc gcg ggg ttg gcg gcg 1603
Cys Arg Tyr Tyr Arg Gly Gly Glu Glu Asn Gly Ala Gly Leu Ala Ala
        410                 415                 420

cac agt tgc ttc ggt gtc gac tac gag ctc tcc ctc att ctg aag gag 1651
His Ser Cys Phe Gly Val Asp Tyr Glu Leu Ser Leu Ile Leu Lys Glu
    425                 430                 435

att aga gtc atc aca gat cag atg cgc aag gac gac gaa gat gcg gac 1699
Ile Arg Val Ile Thr Asp Gln Met Arg Lys Asp Asp Glu Asp Ala Asp
440                 445                 450                 455

att tcg cgc gac tgg aag ttc gcc gcc atg gtc gtg gac aga ctg tgc 1747
Ile Ser Arg Asp Trp Lys Phe Ala Ala Met Val Val Asp Arg Leu Cys
                460                 465                 470

ctt att atc ttt acc ctg ttc aca atc atc gcc acg cta gcc gtg ctg 1795
Leu Ile Ile Phe Thr Leu Phe Thr Ile Ile Ala Thr Leu Ala Val Leu
            475                 480                 485

ctg tcc gcg cca cac atc atg gtg tcg tagcgacccg cccgcttgcg 1842
Leu Ser Ala Pro His Ile Met Val Ser
        490                 495

gatacgcatg cgaaaagttc tgtgataccg cgaatatttg ttaagttgtg atgagcgaag 1902

tggcgcggac ggtgacgccg cggcgtcgga gttgccgccg cctgcctcgc cgcccgcgcc 1962

cccctgtaga cataagttac cgctgactgc caaccctgta cgttcaacaa ataactgccc 2022

atccgactaa cgtcttttat ccccttgaaa aattcagcga ttgtgtaccc ctttcttcca 2082

agaatacaat gacaaatggt cgtcacgctc agtggaatca atcccgtact cttcgcccga 2142

tatttccctt agggtatgtc acgagtttga atgagcggtt ccgtatcaga cgttccgtcc 2202

ccggaacggt cgtcccctgc gataaagtgg cagtacgtgc tatacaggca cttaaggccg 2262

ccacgccacg gcgccgcggt gcgctcgggc cgcgaacccg cgaccctcac cgctgcaagt 2322

ggccacccac tagacaagac tgcggcagaa aatatttgca caaaaacgtc ttccttctta 2382

ccgatgaacg acctgattcg catttaaaat taaactttgt tagaacttct tcgattcttg 2442

aaatctattg tacagtttag agtttgggcg gtgaaacaat ggccctttgt ttccttcttg 2502

ttcgattcca tgaatcgtgg ttataatccc tagttttatt ttcggatata tttgtgtcag 2562

tagctagtat agaactttac aaacaatgtt gattcaattg gtacaggttg tgatatgcct 2622

cgttgtgaac gggtccgata ttgttataaa tggtaaaata cccatggcta tagcttaata 2682

aatcgttcgt taaaagttgt agttaaacaa atattatttt aataaagtca tatctgggtc 2742

ttccggaacg acttttacaa ataattaaat tacatattaa tatcacgttt gtacttcttt 2802

ccatacagtt acagtaattc gtatgctgaa aataatatta gcttgtaaaa ttttcttctt 2862

cgaaaattta ttcaaacaga tgcgaccatc gtttcaaaca tttacatgta atatagaact 2922

cattttataa gatatacaac attttataag tacaagaagt tgtaacatga accggttttt 2982

cgttacatag agggtataac acaaaggtgc ctacatattg acagatgcga agcacgatca 3042

gttgataagc acaggtacac tatatcctga catccgacag tcctgccgct cgtctgccac 3102

actcggaaac attcgacagt tcagtttact gctccgccat catcgattgt taagtttgtt 3162

gttctaactc atcgcattca tttcattcaa aaacattgta aacctctcaa ggggaaaacg 3222

tgttgtaaac agtgagagtg cgcgggtaca accgacacgc gaatgtaccc tcgcaaggct 3282

cctgtaatgt tttcctcttc cgaggtgttg ctgagagtaa tcttagacgg tccgatggaa 3342

gttgcggacc ggatatgatt acaagtcaat gtttttaagt catccgttta tttattgtta 3402

tatcttctta ccattcgcta gaggttgtgt gacgacccgg acggtgggcg ccgcaacccg 3462

cacacgcggg gttccatctt tgtattagat ggaagttgtg cggcatctct ccgtcggcaa 3522

tgggacaacc cgttgtcccc aacatttgtt caattgttag ggttaactct gaattgcact 3582

ttgtttatta aatataaacg aatgaaacaa aaaaaaaaaa aaaaaactcg agagtacttc 3642

tagagcggcc gcgggcccat cgattttcca cccgggtggg gtaccagtaa gtgtaccc 3700

<210> 4
<211> 496
<212> PRT
<213> Heliothis virescens

<400> 4
Met Gly Gly Arg Ala Arg Arg Ser His Leu Ala Ala Pro Ala Gly Leu
1               5                   10                  15

Leu Leu Leu Leu Cys Leu Leu Trp Pro Arg Gly Ala Arg Cys Gly Tyr
            20                  25                  30

His Glu Lys Arg Leu Leu His His Leu Leu Asp His Tyr Asn Val Leu

        35                  40                  45

Glu Arg Pro Val Val Asn Glu Ser Asp Pro Leu Gln Leu Ser Phe Gly
    50                  55                  60

Leu Thr Leu Met Gln Ile Ile Asp Val Asp Glu Lys Asn Gln Leu Leu
65                  70                  75                  80

Ile Thr Asn Ile Trp Leu Lys Leu Glu Trp Asn Asp Met Asn Leu Arg
                85                  90                  95

Trp Asn Thr Ser Asp Phe Gly Gly Val Lys Asp Leu Arg Val Pro Pro
            100                 105                 110

His Arg Leu Trp Lys Pro Asp Val Leu Met Tyr Asn Ser Ala Asp Glu
        115                 120                 125

Gly Phe Asp Ser Thr Tyr Pro Thr Asn Val Val Val Arg Asn Asn Gly
    130                 135                 140

Ser Cys Leu Tyr Val Pro Pro Gly Ile Phe Lys Ser Thr Cys Lys Ile
145                 150                 155                 160

Asp Ile Thr Trp Phe Pro Phe Asp Asp Gln Arg Cys Glu Met Lys Phe
                165                 170                 175

Gly Ser Trp Thr Tyr Asp Gly Tyr Gln Leu Asp Leu Gln Leu Gln Asp
            180                 185                 190

Glu Gly Gly Gly Asp Ile Ser Ser Phe Val Thr Asn Gly Glu Trp Glu
        195                 200                 205

Leu Ile Gly Val Pro Gly Lys Arg Asn Glu Ile Tyr Tyr Asn Cys Cys
    210                 215                 220

Pro Glu Pro Tyr Ile Asp Ile Thr Phe Ala Val Val Ile Arg Arg Lys
225                 230                 235                 240

Thr Leu Tyr Tyr Phe Phe Asn Leu Ile Val Pro Cys Val Leu Ile Ala
                245                 250                 255

Ser Met Ala Leu Leu Gly Phe Thr Leu Pro Pro Asp Ser Gly Glu Lys
            260                 265                 270

Leu Ser Leu Gly Val Thr Ile Leu Leu Ser Leu Thr Val Phe Leu Asn
        275                 280                 285

Met Val Ala Glu Thr Met Pro Ala Thr Ser Asp Ala Val Pro Leu Leu

290                 295                 300

Gly Thr Tyr Phe Asn Cys Ile Met Phe Met Val Ala Ser Ser Val Val
305                 310                 315                 320

Ser Thr Ile Leu Ile Leu Asn Tyr His His Arg His Ala Asp Thr His
                325                 330                 335

Glu Met Ser Asp Trp Ile Arg Cys Val Phe Leu Tyr Trp Leu Pro Trp
            340                 345                 350

Val Leu Arg Met Ser Arg Pro Gly Ser Ala Thr Thr Pro Pro Pro Ala
        355                 360                 365

Arg Val Pro Pro Pro Pro Asp Leu Glu Leu Arg Glu Arg Ser Ser Lys
    370                 375                 380

Ser Leu Leu Ala Asn Val Leu Asp Ile Asp Asp Asp Phe Arg His Pro
385                 390                 395                 400

Gln Ala Gln Gln Pro Gln Cys Cys Arg Tyr Tyr Arg Gly Gly Glu Glu
                405                 410                 415

Asn Gly Ala Gly Leu Ala Ala His Ser Cys Phe Gly Val Asp Tyr Glu
            420                 425                 430

Leu Ser Leu Ile Leu Lys Glu Ile Arg Val Ile Thr Asp Gln Met Arg
        435                 440                 445

Lys Asp Asp Glu Asp Ala Asp Ile Ser Arg Asp Trp Lys Phe Ala Ala
    450                 455                 460

Met Val Val Asp Arg Leu Cys Leu Ile Ile Phe Thr Leu Phe Thr Ile
465                 470                 475                 480

Ile Ala Thr Leu Ala Val Leu Leu Ser Ala Pro His Ile Met Val Ser
                485                 490                 495

<210> 5
<211> 3109
<212> DNA
<213> Heliothis virescens

<220>
<221> CDS
<222> (95)..(1597)

<400> 5
ggcacgagcc ggccgcacgt tgtcccaggc cgcatgagcg cgccggcgtg ctagcgcagc 60

gtgcgcgggt gtggtatgcc cgcgcgtcgc cgct atg gcc cct atg ttg gcg gcc 115
Met Ala Pro Met Leu Ala Ala
1               5

ttg gcg ctg ctg gct ttg ctg ccc gta tcg gag caa ggt cct cac gag 163
Leu Ala Leu Leu Ala Leu Leu Pro Val Ser Glu Gln Gly Pro His Glu
        10                  15                  20

aag aga ctc ctg aac gcg ttg ctg gcg aac tac aac acc ctg gag cga 211
Lys Arg Leu Leu Asn Ala Leu Leu Ala Asn Tyr Asn Thr Leu Glu Arg
    25                  30                  35

ccg gtg gcc aac gag agc gaa ccg cta gag gtc agg ttc ggc ttg acc 259
Pro Val Ala Asn Glu Ser Glu Pro Leu Glu Val Arg Phe Gly Leu Thr
40                  45                  50                  55

ttg cag caa atc att gac gtg gac gag aag aat caa cta ctt ata acc 307
Leu Gln Gln Ile Ile Asp Val Asp Glu Lys Asn Gln Leu Leu Ile Thr
                60                  65                  70

aat ata tgg ctg tcg ttg gag tgg aat gac tac aac ctg agg tgg aac 355
Asn Ile Trp Leu Ser Leu Glu Trp Asn Asp Tyr Asn Leu Arg Trp Asn
            75                  80                  85

gac agc gag tat ggc ggg gtc aag gac ctc agg atc acg ccc aac aag 403
Asp Ser Glu Tyr Gly Gly Val Lys Asp Leu Arg Ile Thr Pro Asn Lys
        90                  95                  100

ttg tgg aag ccg gac gtc ctt atg tat aat agt gct gac gag ggt ttt 451
Leu Trp Lys Pro Asp Val Leu Met Tyr Asn Ser Ala Asp Glu Gly Phe
    105                 110                 115

gac ggg acc tac cag acc aac gtg gtg gtc aga agc ggc ggc agt tgc 499
Asp Gly Thr Tyr Gln Thr Asn Val Val Val Arg Ser Gly Gly Ser Cys
120                 125                 130                 135

ctg tac gtg cca cct ggc ata ttc aag agc aca tgc aag atg gac atc 547
Leu Tyr Val Pro Pro Gly Ile Phe Lys Ser Thr Cys Lys Met Asp Ile
                140                 145                 150

gcg tgg ttt ccc ttc gac gac caa cac tgt gat atg aag ttc ggt agc 595
Ala Trp Phe Pro Phe Asp Asp Gln His Cys Asp Met Lys Phe Gly Ser
            155                 160                 165

tgg aca tat gac ggc aat cag ttg gat ctg gtg cta aaa gat gag gca 643
Trp Thr Tyr Asp Gly Asn Gln Leu Asp Leu Val Leu Lys Asp Glu Ala
        170                 175                 180

ggc ggc gat cta tcg gac ttc ata aca aat ggg gag tgg tat cta ata 691
Gly Gly Asp Leu Ser Asp Phe Ile Thr Asn Gly Glu Trp Tyr Leu Ile
    185                 190                 195

gga atg cca ggc aaa aag aac aca ata aca tac gcg tgc tgc ccc gag 739
Gly Met Pro Gly Lys Lys Asn Thr Ile Thr Tyr Ala Cys Cys Pro Glu
200                 205                 210                 215

ccc tac gtg gac gtc acc ttc acc atc atg ata aga aga cga acc ttg 787
Pro Tyr Val Asp Val Thr Phe Thr Ile Met Ile Arg Arg Arg Thr Leu
                220                 225                 230

tac tac ttc ttc aac ctg atc gtc ccg tgc gtg ctg atc tca tcg atg 835
Tyr Tyr Phe Phe Asn Leu Ile Val Pro Cys Val Leu Ile Ser Ser Met
            235                 240                 245

gca ctc ctc ggc ttc aca ctg cca cca gac tcc gga gag aaa ctc aca 883
Ala Leu Leu Gly Phe Thr Leu Pro Pro Asp Ser Gly Glu Lys Leu Thr
        250                 255                 260

ctt gga gtc act att ctt cta tcg ctg acg gtg ttc ctc aac ctg gta 931
Leu Gly Val Thr Ile Leu Leu Ser Leu Thr Val Phe Leu Asn Leu Val
    265                 270                 275

gcc gag acc ctg cca cag gtc tcc gac gct atc ccc ctg tta ggg acg 979
Ala Glu Thr Leu Pro Gln Val Ser Asp Ala Ile Pro Leu Leu Gly Thr
280                 285                 290                 295

tac ttc aat tgc atc atg ttc atg gta gcg tcg tct gtg gta ctg act 1027
Tyr Phe Asn Cys Ile Met Phe Met Val Ala Ser Ser Val Val Leu Thr
                300                 305                 310

gtg gtg gta ctc aat tac cac cat cga aca gct gat ata cat gaa atg 1075
Val Val Val Leu Asn Tyr His His Arg Thr Ala Asp Ile His Glu Met
            315                 320                 325

cca cag tgg ata aaa tca gta ttc cta caa tgg ttg cca tgg ata ctg 1123
Pro Gln Trp Ile Lys Ser Val Phe Leu Gln Trp Leu Pro Trp Ile Leu
        330                 335                 340

cga atg tcg agg cca ggg aag aag atc acc agg aag act ata atg atg 1171
Arg Met Ser Arg Pro Gly Lys Lys Ile Thr Arg Lys Thr Ile Met Met
    345                 350                 355

aac acg agg atg agg gag ctg gaa ctg aag gag agg tcg tcg aag tcc 1219
Asn Thr Arg Met Arg Glu Leu Glu Leu Lys Glu Arg Ser Ser Lys Ser
360                 365                 370                 375

ttg ctg gcg aat gtt cta gat att gat gat gac ttc aga cac ggc cct 1267
Leu Leu Ala Asn Val Leu Asp Ile Asp Asp Asp Phe Arg His Gly Pro
                380                 385                 390

ccg cct cct aac agt act gcc tcg acc ggg aat ttg gga cct ggg tgc 1315
Pro Pro Pro Asn Ser Thr Ala Ser Thr Gly Asn Leu Gly Pro Gly Cys
            395                 400                 405

tca ata ttc cgc acg gat ttc cgt cgg tcg ttc gtc cgt ccg tcc acg 1363
Ser Ile Phe Arg Thr Asp Phe Arg Arg Ser Phe Val Arg Pro Ser Thr
        410                 415                 420

atg gaa gac gtg ggc ggc ggg ctg ggt agc cac cat cgc gag ctg cac 1411
Met Glu Asp Val Gly Gly Gly Leu Gly Ser His His Arg Glu Leu His
    425                 430                 435

ctc ata ctg aga gag ctg cag ttc atc acg gcc agg atg aag aag gct 1459
Leu Ile Leu Arg Glu Leu Gln Phe Ile Thr Ala Arg Met Lys Lys Ala
440                 445                 450                 455

gat gag gaa gcc gag ctg atc agc gac tgg aag ttt gct gcg atg gtt 1507
Asp Glu Glu Ala Glu Leu Ile Ser Asp Trp Lys Phe Ala Ala Met Val
                460                 465                 470

gtt gat agg ttt tgc ctg ttc gtg ttc aca ctt ttc aca atc atc gcg 1555
Val Asp Arg Phe Cys Leu Phe Val Phe Thr Leu Phe Thr Ile Ile Ala
            475                 480                 485

aca gta gct gtc ctg tta tcg gca ccg cat atc atc gtg caa 1597
Thr Val Ala Val Leu Leu Ser Ala Pro His Ile Ile Val Gln
        490                 495                 500

tgaaccaacc actgagccgg caactccggc gcatgaatga gagaaataat tattagatcg 1657

ccgatttgta attataattg ataatgtaat taaattaaat acgtggttga aacgcacacg 1717

tctccataac aaagtcttaa gacattaaat tatgataaat ttacatattg tagttaagtc 1777

gagtgttgat ggaaatttta gccggcgcaa ggagtttcgt gaaggtctgt atatattttt 1837

tcttattgtt gtatattgta tcgttgttca tgttttcttt caggaagtga gctttgtact 1897

gtttgtttct tcgatggcag gtgcacttca gttcaggctg aaatttccat taacatttat 1957

ttaaacaaat gtgatgttga ctaggatgtt atacagataa atgttgacgt gtataatttg 2017

ttaaaataaa caatattaat tactattact aaacgatatt ataaacgaag tactaacgag 2077

ggttacttta atgggaagaa cgctaagctg gcacagagtt gcattaattt gaaaaaagaa 2137

attacggaaa aaagtttatt gaaaattgaa ctttttggaa ggaaagtaac gtttgatcaa 2197

aaaagtttgt aaaacgaaag ttcggttctg cgccaatact ggaattaaaa ttctcgtaaa 2257

tattagggaa aagaaggtcc tttaaaacaa aagatttgaa ccggcatcct ttttacaagt 2317

aatgagggat cacagatgat gacaaaaaac cttagggtat ataagtaatg tacataatgg 2377

atcaaatatc ggtagagtca agaatagtta acgatttaag attattccat tcgatattaa 2437

aattcgatta gcgattgtcg ctgcgtctac tttgatacat atcgatttga atcgatattg 2497

tataaattta gatagatcgg acattagtaa tgagtatgga cgttttaatt tttaaaaaag 2557

aatgtactac gaagattaaa tccaggaatt gttaaacagt tatggaattg ataagaaatc 2617

aacaattaat acggaaccaa aggtagacta ggtgtagcat caggagattg aattaaaaca 2677

taaattagga ccgacttaaa tggaacttgc gagtgtattg ataacttttt aatttaaaaa 2737

ctcattgtcg attaaatgga gaataacttt tgatctctcg tatcgataaa tgctcactta 2797

actatcgata gcgtaatatt ataactgtta gtatatcgat atgggagtaa gtcactagca 2857

tcagaaatag tcattaatta ggaatcggtt tgtgttaatg ttatgcttag cgaaaatatt 2917

acaatgctgt tgatatcact aaccatcacg taaccatatt gataaaatgt aaatacagaa 2977

tattgcggtg tgtatttgta tataaatttt agaaaaaaaa aaaaaaaaaa aactcgagag 3037

tacttctaga gcggccgcgg gcccatcgat tttccacccg ggtggggtac caggtaagtg 3097

tacccaattc gc 3109

<210> 6
<211> 501
<212> PRT
<213> Heliothis virescens

<400> 6
Met Ala Pro Met Leu Ala Ala Leu Ala Leu Leu Ala Leu Leu Pro Val

1                   5                   10                  15

Ser Glu Gln Gly Pro His Glu Lys Arg Leu Leu Asn Ala Leu Leu Ala
                20                  25                  30

Asn Tyr Asn Thr Leu Glu Arg Pro Val Ala Asn Glu Ser Glu Pro Leu
            35                  40                  45

Glu Val Arg Phe Gly Leu Thr Leu Gln Gln Ile Ile Asp Val Asp Glu
    50                  55                  60

Lys Asn Gln Leu Leu Ile Thr Asn Ile Trp Leu Ser Leu Glu Trp Asn
65                  70                  75                  80

Asp Tyr Asn Leu Arg Trp Asn Asp Ser Glu Tyr Gly Gly Val Lys Asp
                85                  90                  95

Leu Arg Ile Thr Pro Asn Lys Leu Trp Lys Pro Asp Val Leu Met Tyr
            100                 105                 110

Asn Ser Ala Asp Glu Gly Phe Asp Gly Thr Tyr Gln Thr Asn Val Val
        115                 120                 125

Val Arg Ser Gly Gly Ser Cys Leu Tyr Val Pro Pro Gly Ile Phe Lys
    130                 135                 140

Ser Thr Cys Lys Met Asp Ile Ala Trp Phe Pro Phe Asp Asp Gln His
145                 150                 155                 160

Cys Asp Met Lys Phe Gly Ser Trp Thr Tyr Asp Gly Asn Gln Leu Asp
                165                 170                 175

Leu Val Leu Lys Asp Glu Ala Gly Gly Asp Leu Ser Asp Phe Ile Thr
            180                 185                 190

Asn Gly Glu Trp Tyr Leu Ile Gly Met Pro Gly Lys Lys Asn Thr Ile
        195                 200                 205

Thr Tyr Ala Cys Cys Pro Glu Pro Tyr Val Asp Val Thr Phe Thr Ile
    210                 215                 220

Met Ile Arg Arg Arg Thr Leu Tyr Tyr Phe Phe Asn Leu Ile Val Pro
225                 230                 235                 240

Cys Val Leu Ile Ser Ser Met Ala Leu Leu Gly Phe Thr Leu Pro Pro
                245                 250                 255

Asp Ser Gly Glu Lys Leu Thr Leu Gly Val Thr Ile Leu Leu Ser Leu

            260                 265                 270

Thr Val Phe Leu Asn Leu Val Ala Glu Thr Leu Pro Gln Val Ser Asp
        275                 280                 285

Ala Ile Pro Leu Leu Gly Thr Tyr Phe Asn Cys Ile Met Phe Met Val
    290                 295                 300

Ala Ser Ser Val Val Leu Thr Val Val Val Leu Asn Tyr His His Arg
305                 310                 315                 320

Thr Ala Asp Ile His Glu Met Pro Gln Trp Ile Lys Ser Val Phe Leu
                325                 330                 335

Gln Trp Leu Pro Trp Ile Leu Arg Met Ser Arg Pro Gly Lys Lys Ile
            340                 345                 350

Thr Arg Lys Thr Ile Met Met Asn Thr Arg Met Arg Glu Leu Glu Leu
        355                 360                 365

Lys Glu Arg Ser Ser Lys Ser Leu Leu Ala Asn Val Leu Asp Ile Asp
    370                 375                 380

Asp Asp Phe Arg His Gly Pro Pro Pro Pro Asn Ser Thr Ala Ser Thr
385                 390                 395                 400

Gly Asn Leu Gly Pro Gly Cys Ser Ile Phe Arg Thr Asp Phe Arg Arg
                405                 410                 415

Ser Phe Val Arg Pro Ser Thr Met Glu Asp Val Gly Gly Gly Leu Gly
            420                 425                 430

Ser His His Arg Glu Leu His Leu Ile Leu Arg Glu Leu Gln Phe Ile
        435                 440                 445

Thr Ala Arg Met Lys Lys Ala Asp Glu Glu Ala Glu Leu Ile Ser Asp
    450                 455                 460

Trp Lys Phe Ala Ala Met Val Val Asp Arg Phe Cys Leu Phe Val Phe
465                 470                 475                 480

Thr Leu Phe Thr Ile Ile Ala Thr Val Ala Val Leu Leu Ser Ala Pro
                485                 490                 495

His Ile Ile Val Gln
            500

## Beschreibung

Die Erfindung betrifft insbesondere Nukleinsäuren, die für Acetylcholinrezeptor-Untereinheiten von Insekten kodieren.

Nikotinische Acetylcholinrezeptoren sind ligandengesteuerte Ionenkanäle, die eine Rolle bei der Neurotransmission im Tierreich spielen. Die Bindung von Acetylcholin oder anderen Agonisten an den Rezeptor verursacht eine vorübergehende Öffnung des Kanals und gestattet den Durchstrom von Kationen. Man nimmt an, daß ein Rezeptor aus fünf Untereinheiten besteht, die sich um eine Pore gruppieren. Jede dieser Untereinheiten ist ein Protein, das aus einem extrazellulären N-terminalen Teil besteht, gefolgt von drei Transmembranregionen, einem intrazellulären Teil, sowie einer vierten Transmembranregion und einem kurzen extrazellulären C-terminalen Teil (Changeux et al. 1992).

Acetylcholinrezeptoren sind vor allem in Wirbeltieren gut untersucht. Anhand ihrer anatomischen Lokalisierung und ihrer funktionellen Eigenschaften (Leitungseigenschaften des Kanals, Desensibilisierung, Sensitivität gegenüber Agonisten und Antagonisten, sowie gegenüber Toxinen wie z.B. α-Bungarotoxin) lassen sich hier drei Gruppen unterscheiden. Die Einteilung korreliert mit der molekularen Zusammensetzung der Rezeptoren. Es gibt heterooligomere Rezeptoren mit der Untereinheitenzusammensetzung α₂βγδ, die im Muskel vorkommen (Noda et al. 1982, Claudio et al. 1983, Devillers-Thiery et al. 1983, Noda et al. 1983a, b), heterooligomere Rezeptoren, die Untereinheiten aus der Gruppe α2 - α6 und β2 - β4 enthalten, und die im Nervensystem vorkommen (Wada et al. 1988, Schoepfer et al. 1990, Cockcroft et al. 1991, Heinemann et al. 1997), sowie homooligomere Rezeptoren, die Untereinheiten aus der Gruppe α7 - α9 enthalten, und die ebenfalls im Nervensystem vorkommen (Lindstrom et al. 1997, Elgoyhen et al. 1997). Diese Einteilung wird auch durch eine Betrachtung der Verwandschaft der Gensequenzen der verschiedenen Untereinheiten gestützt. Typischerweise sind die Sequenzen funktionell homologer Untereinheiten verschiedener Spezies ähnlicher als Sequenzen von Untereinheiten aus verschiedenen Gruppen, aber der gleichen Spezies. So weist z.B. die muskuläre α-Untereinheit der Ratte 78 % identische und 84 % ähnliche Aminosäuren auf mit der des elektrischen Rochens Torpedo californica, aber nur 48 % Identität und 59 % Ahnlichkeit mit der α2-Untereinheit (heterooligomer, neuronal) der Ratte, und 36 % Identität und 45 % Ähnlichkeit mit der α7-Untereinheit (homooligomer, neuronal) der Ratte. Weiterhin sind die Gensequenzen aller bekannten Acetylcholinrezeptor-Untereinheitenen nicht nur untereinander in gewissem Maße ähnlich, sondern auch mit denen einiger anderer ligandengesteuerter lonenkanäle (z.B. den Serotoninrezeptoren vom Typ 5HT₃, den GABA-gesteuerten Chloridkanälen, den Glycin-gesteuerten Chloridkanälen). Man geht daher davon aus, daß alle diese Rezeptoren von einem gemeinsamen Vorläufer abstammen und ordnet sie in eine Supergenfamilie ein (Ortells et al. 1995).

In Insekten ist Acetylcholin der wichtigste exzitatorische Neurotransmitter des zentralen Nervensystems. Dementsprechend lassen sich Acetylcholinrezeptoren an Präparaten zentraler Ganglien aus Insekten elektrophysiologisch nachweisen. Der Nachweis gelingt sowohl an post- als auch an präsynaptischen Nervenendigungen, sowie an den Zellkörpern von Interneuronen, Motorneuronen und modulatorischen Neuronen (Breer et al. 1987, Buckingham et al. 1997). Unter den Rezeptoren gibt es solche, die durch a-Bungarotoxin inhibiert werden, und solche, die insensitiv sind (Schloß et al. 1988). Die Acetylcholinrezeptoren sind außerdem der molekulare Angriffspunkt wichtiger natürlicher (z.B. Nikotin) und synthetischer Insektizide (z.B. Chloronikotinyle).

Die Gensequenz einer Anzahl von nikotinischen Acetylcholinrezeptoren der Insekten ist bereits bekannt. So sind in Drosophila melanogaster die Sequenzen fünf verschiedener Untereinheiten beschrieben (Bossy et al. 1988, Hermanns-Borgmeyer et al. 1986, Sawruk et al. 1990a, 1990b, Schulz et al. unveröffentlicht, EMBL accession number Y15593), in Locusta migratoria ebenfalls fünf (Stetzer et al. unveröffentlicht, EMBL accession numbers AJ000390 - AJ000393), in Schistocerca gregaria eine (Marshall et al. 1990), in Myzus persicae zwei (Sgard et al. unveröffentlicht, EMBL accession number X81887 und X81888), in Manduca sexta eine Sequenz (Eastham et al. 1997). Zudem ist eine Reihe von partiellen Gensequenzen aus Drosophila melanogaster als sog. expressed sequence tags charakterisiert worden (Genbank accession numbers AA540687, AA698155, AA697710, AA697326). Die hohe Ähnlichkeit einzelner Sequenzen mit solchen aus anderen Insekten legt nahe, daß es sich bei diesen Untereinheiten um fünktionelle Homologe handelt.

Es ist von großer praktischer Bedeutung, beispielsweise für die Suche nach neuen Insektiziden, neue Untereinheiten von Acetylcholinrezeptoren aus Insekten bereitzustellen, wobei besonders solche von Interesse sind, die sich von den bekannten stärker unterscheiden, als dies zwischen funktionellen Homologen der Fall ist.

Der vorliegenden Erfindung liegt somit insbesondere die Aufgabe zugrunde, Nukleinsäuren zur Verfügung zu stellen, die neue Acetylcholinrezeptor-Untereinheiten von Insekten kodieren.

Die Aufgabe wird gelöst durch die Bereitstellung von Nuldeinsäuren umfassend eine Sequenz ausgewählt aus
(a) den Sequenzen gemäß SEQ ID NO: 1, SEQ ID NO: 3 oder SEQ ID NO: 5,
(b) zumindest 14 Basenpaare langen Teilsequenzen der unter (a) definierten Sequenzen,
(c) Sequenzen, welche an die unter (a) definierten Sequenzen hybridisieren in 2 x SSC bei 60°C, bevorzugt in 0,5 x SSC bei 60°C, besonders bevorzugt in 0,2 x SSC bei 60°C (Sambrook et al. 1989),
(d) Sequenzen, welche eine zumindest 70 %ige Identität zu den unter (a) definierten Sequenzen zwischen Position 1295 und Position 2195 aus SEQ ID NO: 1 oder zwischen Position 432 und Position 1318 aus SEQ ID NO: 3 oder zwischen Position 154 und Position 1123 aus SEQ ID NO: 5 aufweisen,
(e) Sequenzen, welche zu den unter (a) definierten Sequenzen komplementär sind und
(f) Sequenzen, welche aufgrund der Degeneration des genetischen Codes für dieselbe Aminosäuresequenz kodieren wie die unter (a) bis (d) definierten Sequenzen.

Der Grad der Identität der Nukleinsäuresequenzen wird vorzugsweise bestimmt mit Hilfe des Programms GAP aus dem Programmpaket GCG, Version 9.1 unter Standardeinstellungen (Devereux et al. 1984).

Die vorliegende Erfindung begründet sich auf den überraschenden Befund, daß Insekten Gene besitzen, die für Untereinheiten von insbesondere homooligomeren Acetylcholinrezeptoren kodieren.

Gegenstand der Erfindung sind weiterhin Vektoren, die zumindest eine der erfindungsgemäßen Nukleinsäuren enthalten. Als Vektoren können alle in molekularbiologischen Laboratorien verwendete Plasmide, Phasmide, Cosmide, YACs oder künstliche Chromosomen verwendet werden. Für die Expression der erfindungsgemäßen Nukleinsäuren können diese mit üblichen regulatorischen Sequenzen verknüpft werden. Die Auswahl solcher regulatorischen Sequenzen ist davon abhängig, ob zur Expression pro- oder eukaryotische Zellen bzw. Zellfreie Systeme verwendet werden. Besonders bevorzugt als Expressionskontrollsequenz sind z.B. der frühe oder späte Promotor des SV40 oder des Adenovirus, des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Zur Expression der erfindungsgemäßen Nukleinsäuren können diese in geeignete Wirtszellen eingebracht werden. Als Wirtszellen eignen sich sowohl prokaryotische Zellen, vorzugsweise E.coli, als auch eukaryotische Zellen, vorzugsweise Säuger- oder Insektenzellen, Weitere Beispiele für geeignete einzellige Wirtzellen sind: Pseudomonas, Bacillus, Streptomyces, Hefen, HEK-293, Schneider S2, CHO-, COS1-, COS7-, Zellen, Pflanzenzellen in Zellkultur sowie Amphibienzellen, insbesondere Oocyten.

Gegenstand der vorliegenden Erfindung sind auch die Polypeptide, die von den erfindungsgemäßen Nukleinsäuren kodiert werden, sowie die daraus aufgebauten Acetylcholinrezeptoren, bevorzugt homooligomere Acetylcholinrezeptoren.

Zur Herstellung der Polypeptide, die von den erfindungsgemäßen Nukleinsäuren kodiert werden, können Wirtszellen, die zumindest eine der erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden.

Weiterhin sind Antikörper Gegenstand der Erfindung, die spezifisch an die vorstehend genannten Polypeptide bzw. Rezeptoren binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Acetylcholinrezeptor-Polypeptids oder eines Fragments davon und durch nachfolgende Gewinnung dieses Antikörpers. Weiterhin läßt sich in an sich bekannter Weise eine immortalisierte Zellinie erhalten, die monoklonale Antikörper produziert. Die Antikörper können gegebenenfalls mit einem Nachweisreagenz markiert sein, Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen.

Die erfindungsgemäßen Nukleinsäuren können insbesondere zur Herstellung transgener Invertebraten verwendet werden. Diese können in Testsysteme eingesetzt werden, die auf einer vom Wildtyp abweichenden Expression der erfindungsgemäßen Rezeptoren oder Varianten hiervon basieren. Ferner fallen hierunter sämtliche transgenen Invertebraten, bei denen durch die Modifikation anderer Gene oder Genkontrollsequenzen (Promotoren) eine Veränderung der Expression der erfindungsgemäßen Rezeptoren oder deren Varianten eintritt.

Die Herstellung der transgenen Invertebraten erfolgt beispielsweise in Drosophila melanogaster durch P-Element vermittelten Gentransfer (Hay et al., 1997) oder in Caenorhabditis elegans durch Transposon vermittelten Gentransfer (z.B. durch Tc1, Plasterk, 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans, sowie deren transgene Nachkommen. Vorzugsweise enthalten die transgenen Invertebraten die erfindungsgemäßen Rezeptoren in einer vom Wildtyp abweichenden Form.

Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken zu durchsuchen. Klone, an die die markierten Oligonukleotide hybridisieren ("positive Klone"), werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Die erfindungsgemäßen Nukleinsäuren können zur Isolierung und Charakterisierung der regulatorischen Regionen, die natürlicherweise benachbart zu der kodierenden Region vorkommen, verwendet werden. Solche regulatorischen Regionen sind somit ebenfalls Gegenstand der vorliegenden Erfindung.

Mit Hilfe der erfindungsgemäßen Nukleinsäuren können neue Wirkstoffe für den Pflanzenschutz identifiziert werden, z.B. Verbindungen, welche als Modulatoren, insbesondere als Agonisten oder Antagonisten, die Leitungseigenschaften der erfindungsgemäßen Acetylcholinrezeptoren verändern. Dazu wird ein rekombinantes DNA-Molekül, das zumindest ein erfindungsgemäßes Nukleinsäuremolekül umfaßt, in eine geeignete Wirtszelle eingebracht. Die Wirtzelle wird in Gegenwart einer Verbindung oder einer Probe, welche eine Vielzahl von Verbindungen umfaßt, unter Bedingungen kultiviert, die die Expression der erfindungsgemäßen Rezeptoren erlauben. Eine Veränderung der Rezeptoreigenschaften kann - wie nachstehend in Beispiel 2 beschrieben - detektiert werden. Auf diese Weise ist es möglich, insektizide Substanzen aufzufinden.

Die erfindungsgemäßen Nukleinsäuren ermöglichen auch das Auffinden von Verbindungen, die an die erfindungsgemäßen Rezeptoren binden. Diese können ebenfalls als Insektizide auf Pflanzen angewandt werden. Beispielsweise werden Wirtszellen, die die erfindungsgemäßen Nukleinsäuren enthalten und die entsprechenden Rezeptoren bzw. Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Unter Verwendung von Wirtszellen oder transgenen Invertebraten, die die erfindungsgemäßen Nukleinsäuren enthalten, ist es auch möglich, Substanzen aufzufinden, welche die Expression der Rezeptoren verändern.

Die vorstehend beschriebenen erfindungsgemäßen Nukleinsäuren, Vektoren und regulatorischen Regionen können außerdem zum Auffinden von Genen verwendet werden, die für Polypeptide kodieren, welche am Aufbau von funktionell ähnlichen Acetylcholinrezeptoren in Insekten beteiligt sind. Unter funktionell ähnlichen Rezeptoren werden gemäß der vorliegenden Erfindung Rezeptoren verstanden, die Polypeptide umfassen, welche sich zwar hinsichtlich der Aminosäuresequenz von den hierin beschriebenen Polypeptiden unterscheiden, aber im wesentlichen dieselben Funktionen haben.

### Erläuterungen zum Sequenzprotokoll und zu den Figuren:

SEQ ID NO: 1 zeigt die Nukleotidsequenz der isolierten Da7 cDNA, beginnend mit Position 1 und endend mit Position 2886. SEQ ID NO: 1 und SEQ ID NO: 2 zeigen ferner die Aminosäuresequenzen des von der Da7 cDNA Sequenz abgeleiteten Proteins.
SEQ ID NO: 3 zeigt die Nukleotidsequenz der isolierten Hva7-1 cDNA, beginnend mit Position 1 und endend mit Position 3700. SEQ ID NO: 3 und SEQ ID NO: 4 zeigen ferner die Aminosäuresequenzen des von der Hva7-1 cDNA Sequenz abgeleiteten Proteins.
SEQ ID NO: 5 zeigt die Nukleotidsequenz der isolierten Hva7-2 cDNA, beginnend mit Position 1 und endend mit Position 3109. SEQ ID NO: 5 und SEQ ID NO: 6 zeigen ferner die Aminosäuresequenzen des von der Hva7-2 cDNA Sequenz abgeleiteten Proteins.

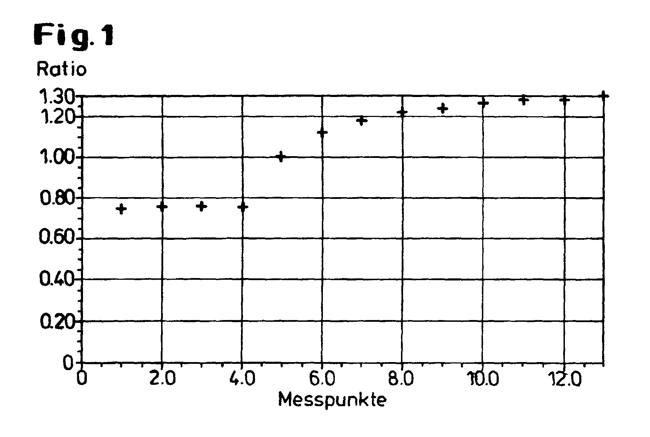

Figur 1 zeigt den Anstieg des intrazellulären Calciums in gentechnisch veränderten Zellen gemäß Beispiel 2 nach Gabe von Nikotin. Zellen wurden mit Fura-2-acetoxymethylester (5 - 10 µM in serumfreiem Minimal Essentiellem Medium mit 1 % Rinderserumalbumin and 5 mM Calciumchlorid) beladen, mit N-(2-Hydroxyethyl)piperazine-N'-(2-ethanesulfonic acid) (5 mM HEPES) gepufferter Tyrode-Lösung gewaschen und unter einem Fluoreszenzmikroskop (Nikon Diaphot) abwechselnd mit Licht der Wellenlänge 340 nm und 380 nm bestrahlt. Ein Meßpunkt entspricht einem Paar von Videobildern bei beiden Wellenlängen (Belichtungszeit pro Bild 100 ms). Der Zeitabstand von zwei Meßpunkten beträgt 3 s. Nach Aufnahme von 8 Bildern (Meßpunkt 4.0) wurde Nikotin auf eine Endkonzentration von 500 µM zugegeben, und die Meßreihe fortgesetzt. Die Fluoreszenzintensität der Zellen bei Bestrahlung mit Licht der Wellenlänge 380 nm wurde durch die entsprechende Intensität bei 340 nm geteilt und so das Verhältnis ("Ratio") gebildet.

### Beispiele:

### Beispiel 1

### Isolierung der beschriebenen Polynukleotidsequenzen

Die Manipulation von Polynukleotiden erfolgte nach Standardmethoden der rekombinanten DNA Technologie (Sambrook, et al., 1989). Die bioinformatische Bearbeitung von Nukleotid- und Proteinsequenzen erfolgten mit dem Programmpaket GCG Version 9.1 (GCG Genetics Computer Group, Inc., Madison Wisconsin, USA).

### Partielle Polynukleotidsequenzen

Aus Proteinsequenzen von Genen, bei denen ihre Fähigkeit homooligomere Acetylcholinrezeptoren auszubilden bekannt war, wurden durch Sequenzvergleiche ("Clustalw") Bereiche identifiziert, aus denen durch Rücktranslation der Codons degenerierte Oligonukleotide abgeleitet wurden. Insgesamt wurden 5 solcher Oligonukleotidpaare für die Polymerasekettenreaktion (PCR) ausgewählt. Nur eine Kombination (vide infra) ergab ein Produkt sowohl aus Heliothis-cDNA als auch aus Drosophila-cDNA.

RNA wurde aus gesamten Heliothis virescens-Embryonen (kurz vor Schlupf) mittels Trizol-Reagenz (Gibco BRL, nach Angaben des Herstellers) isoliert. In gleicher Weise wurde mit Drosophila-Embryonen (24 h bei 25°C) verfahren. 10 µg dieser RNAs wurden in eine cDNA-Erststrangsynthese (Superscript Präamplifizierungssystem für die cDNA-Erststrangsynthese, Gibco BRL, nach Angaben des Herstellers, 45°C Reaktionstemperatur) eingesetzt.

Anschließend wurden jeweils 1/100 der o.g. Erststrang-cDNA in eine Polymerasekettenreaktion (PCR) mit den Oligonukleotiden alpha7-1s: (5'-GAYGTIGAYGARAARAAYCA-3') und alpha7-2a: (5'-CYYTCRTCIGCRCTRTTRTA-3') eingesetzt (Taq DNA Polymerase, rekombinant, Gibco BRL). Die PCR-Parameter waren wie folgt: Hva7-1 und Hva-7-2: 94°C, 2 mm; 35 mal (94°C, 45 s; 50°C, 30 s; 72°C, 60 s) sowie Da7: 96°C, 2 min; 35 mal (96°C, 45 s; 50°C, 30 s; 72°C, 60 s). Hieraus ergab sich jeweils eine im Agarosegel (1 %) erkennbare Bande von ca. 0,2 kb sowohl bei Drosophila-cDNA als auch bei Heliothis-cDNA. Nach Subkionierung der DNA-Fragmente mittels SrfScript (Stratagene) und Bestimmung der DNA-Sequenz, zeigte sich, daß aus Heliothis-cDNA zwei verschiedene DNA-Fragmente amplifiziert worden waren; diese waren 228-11 = Hva7-1(partiell, mit 165 bp) und 228-8 = Hva7-2 (partiell, mit 171 bp). Aus Drosophila-cDNA wurde nur ein DNA-Fragment isoliert; dieses war 248-5 = Da7(partiell, mit 150 bp).

### Isolierung von poly A enthaltender RNA aus Heliothis virescens-Gewebe und Konstruktion der cDNA-Bibliotheken

Die RNA für die cDNA-Bibliothek I wurde aus gesamten Heliothis virescens-Embryonen (kurz vor Schlupf) mittels Trizol-Reagenz (Gibco BRL, nach Angaben des Herstellers) isoliert. Die RNA für die cDNA-Bibliothek II wurde aus gesamten Kopfganglien von 500 Heliothis virescens-Larven (Stadien 4-5) mittels Trizol-Reagenz (Gibco BRL, nach Angaben des Herstellers) isoliert. Aus diesen RNAs wurden nun die poly A enthaltenden RNAs durch Reinigung über Dyna Beads 280 (Dynal) isoliert. 5 µg dieser poly A enthaltenden RNAs wurden anschließend in die Konstruktion der cDNA-Bibliotheken I und II mit dem λ-ZAPExpress Vektor eingesetzt (cDNA Synthesis Kit, ZAP-cDNA Synthesis Kit und ZAP-cDNA Gigapack III Gold Cloning Kit, alle Stratagene). In Abweichung von den Angaben des Herstellers wurde zur cDNA-Synthese die Reverse Transkriptase Superscript (Gibco BRL) bei einer Synthesetemperatur von 45°C verwendet. Außerdem wurde auf die Zugabe radioaktiv markierter Desoxynukleosidtriphosphate verzichtet. Desweiteren wurden die synthetisierten cDNAs nicht über das im Kit enthaltene Gelfiltrationsmedium, sondern über Size Sep 400 Spun Columns (Pharmacia) fraktioniert.

### Vollständige Polynukleotidsequenzen

Mit Ausnahme der ersten Screening Runde bei der Isolierung des Hva7-1 Klons, erfolgten alle Screens mit Hilfe des DIG Systems (alle Reagenzien und Verbrauchsmaterialien Boehringer Mannheim, nach Angaben im "The DIG System User's Guide for Filter Hybridization", Boehringer Mannheim). Die eingesetzten DNA-Sonden wurden durch PCR mittels Digoxygenin markiertem dUTP präpariert, Die Hybridisierungen erfolgten in DIG Easy Hyb (Boehringer Mannheim) bei 42°C über Nacht. Der Nachweis markierter DNA auf Nylonmembranen geschah durch Chemolumineszenz (CDP-Star, Boehringer Mannheim) unter Verwendung von Röntgenfilmen (Hyperflim MP, Amersham). Die isolierten Genbankplasmide wurden zur Identifikation mittels T3 und T7 Primer ansequenziert (ABI Prism Dye Terminator Cycle Sequencing Kit, ABI, mit ABI Prism 310 Genetic Analyzer). Die Bestimmung der vollständigen Polynukleotidsequenzen in Hva7-1, Hva7-2 und Da7 erfolgte durch Primer Walking mittels Cycle Sequencing als Auftragssequenzierung bei der Firma Qiagen, Hilden.

### a. Isolierung des Da7 Klons

10⁶ Phagen einer Drosophila melanogaster-cDNA-Bibliothek in λ Phagen (Canton-S embryo, 2-14 Stunden, in Uni-ZAP XR Vektor, Stratagene) wurden einem Screening mit DIG markiertem 248-5 als Sonde unterzogen (nach Angaben des Herstellers Stratagene). Die maximale Stringenz beim Waschen der Filter betrug: 0,2 x SSC; 0,1 % SDS; 42°C; 2 x 15 min. Es konnte ein Klon isoliert werden(Klon 432-1), dessen Insert eine Größe von 2940 bp aufwies (Da7, SEQ ID NO: 1). Der größte offene Leserahmen dieser Sequenz beginnt bei der Position 372 der dargestellten Sequenz und endet bei Position 1822. Das hieraus abgeleitete Polypeptid umfaßt 770 Aminosäuren (SEQ ID NO: 2) und besitzt ein errechnetes Molekulargewicht von 87,01 kD.

### b. Isolierung des Hva7-1 Klons

In das Screening gingen 10⁶ Phagen der Heliothis virescens-Embryo-cDNA-Bibliothek (Bibliothek I) ein. Die erste von drei Screening Runden fand unter Verwendung α-³²P markierter 228-11 DNA als Sonde statt. Die Hybridisierung der Sonde an die Filter erfolgte in Quickhyb (Stratagene) bei 68°C für eine Stunde. Anschließend wurden die Filter zwei mal je 15 min bei Raumtemperatur in 2 x SSC; 0,1 %SDS und 2 mal je 30 min bei 42°C in 0,1xSSC; 0,1 % SDS gewaschen. Die Detektion hybridisierter Sonden erfolgte durch Autoradiographie mit XR Röntgenfilmen (Kodak) unter Verwendung von Verstärkerfolien (Amersham) bei -80°C über Nacht. Die zwei weiteren Screening Runden erfolgten unter Verwendung des DIG Systems (Boehringer Mannheim).

Der in diesem Screen isolierte Klon 241-5 enthielt ein Insert von 3630 bp. Dieses Insert (Hva7-1, SEQ ID NO: 3) besitzt einen längsten offenen Leserahmen, der bei Position 335 der dargestellten Nukleinsäuresequenz beginnt und bei Position 1821 endet. Das hieraus abgeleitete Polypeptid umfaßt 496 Aminosäuren (SEQ ID NO: 4) und besitzt ein errechnetes Molekulargewicht von 56,36 kD.

### c. Isolierung des Hva7-2 Klons

In das Screening gingen 10⁶ Phagen der Heliothis virescens-Ganglien-cDNA-Bibliothek (Bibliothek II) ein. Als Sonde wurde Dig markierte 228-8 DNA verwendet. Die maximale Stringenz beim Waschen der Filter betrug: 0,1 x SSC; 0,1% SDS; 42°C; 2 x 15 min.

Der in diesem Screen isolierte Klon 241-5 enthielt ein Insert von 3630 bp. Dieses Insert (Hva7-2, SEQ ID NO: 5) besitzt einen längsten offenen Leserahmen, der bei Position 95 der dargestellten Nukleinsäuresequenz beginnt und bei Position 1598 endet. Das hieraus abgeleitete Polypeptid umfaßt 501 Aminosäuren (SEQ ID NO: 6) und besitzt ein errechnetes Molekulargewicht von 56,71 kD.

### Beispiel 2

### Generierung der Expressionskonstrukte

### a. Da7

Mittels Polymerasekettenreaktion (PCR) wurde der Sequenzbereich von Position 372 bis Position 2681 aus SEQ ID NO: 1 amplifiziert. Hierzu wurden Desoxyoligonukleotide mit den Sequenzen
GCGAATTCACCACCATGAAAAATGCACAACTG sowie CGAGACAATAATATGTGGTGCCTCGAG verwendet. Als DNA-Polymerase wurde die Pfu-Polymerase von Stratagene nach Angaben des Herstellers verwendet. Nach erfolgter Amplifikation wurde das generierte Stück mit den Restriktionsendonukleasen Eco RI und Xho I verdaut und in einen ebenfalls Eco RI und XhoI verdauten Vektor pcDNA3.1/Zeo (Invitrogen) einkloniert.

### b. Hva7-1

Mittels Polymerasekettenreaktion (PCR) wurde der Sequenzbereich von Position 335 bis Position 1822 aus SEQ ID NO: 3 amplifiziert. Hierzu wurden Desoxyoligonukleotide mit den Sequenzen
GCAAGCTTACCACCATGGGAGGTAGAGCTAGACGCTCGCAC sowie GCCTCGAGCGACACCATGATGTGTGGCGC verwendet. Als DNA-Polymerase wurde die Pfu-Polymerase von Stratagene nach Angaben des Herstellers verwendet. Nach erfolgter Amplifikation wurde das generierte Stück mit den Restriktionsendonukleasen HindIII und Xho I verdaut und in einen ebenfalls HindIII und XhoI verdauten Vektor pcDNA3.1/Zeo (Invitrogen) einkloniert.

### c. Hva7-2

Mittels Polymerasekettenreaktion (PCR) wurde der Sequenzbereich von Position 95 bis Position 1597 aus SEQ ID NO: 5 amplifiziert. Hierzu wurden Desoxyoligonukleotide mit den Sequenzen
GCAAGCGCCGCTATGGCCCCTATGTTG sowie TTGCACGATGATATGCGGTGCCTCGAGCG verwendet. Als DNA-Polymerase wurde die Pfu-Polymerase von Stratagene nach Angaben des Herstellers verwendet. Nach erfolgter Amplifikation wurde das generierte Stück mit den Restriktionsendonukleasen HindIII und Xho I verdaut und in einen ebenfalls HindIII und XhoI verdauten Vektor pcDNA3.1/Zeo (Invitrogen) einkloniert.

### d.Hva7-1 / 5HT₃ sowie Hva7-2 / 5HT₃ Chimären

Durch die Methode der Overlap Extension (Jespersen et al. 1997) wurde jeweils der Bereich von Position 335 bis Position 1036 aus SEQ ID NO: 3 (Hva7-1/5HT₃ Chimäre) sowie der Bereich von Position 95 bis Position 763 aus SEQ ID NO: 5 (Hva7-2/5HT₃ Chimäre) mit dem Bereich von Position 778 bis Position 1521 aus der Mus museulus 5-HT₃ Rezeptor-cDNA (Sequenz in EMBL Datenbank: M774425) fusioniert. Die beiden Fragmente wurden anschließend mittels TA Cloning (Invitrogen, nach Angaben des Herstellers) in den pcDNA3.1/Zeo Vektor kloniert. Konstrukte mit korrekter Orientierung der beiden Fragmente im Vektor wurden durch Sequenzierung mit dem T7 Primer (Invitrogen) identifiziert.

### Zellkultur und Gentransfer

HEK293-Zellen, die die α-Untereinheit eines L-Typ Ca-Kanals exprimieren (Zong et al. 1995, Stetzer et al. 1996), wurden in Dulbeccos Modified Eagles Medium und 10 % foetalem Kälberserum bei 5 % CO₂ und 20°C bis 37°C kultiviert. Für den Gentransfer wurde FuGENE 6 (Boehringer Mannheim GmbH, Mannheim, Deutschland) nach Angaben des Herstellers verwendet. 24 h bis 48 h nach dem Gentransfer wurden die Zellen in verschiedenen Dichten in Mikrotiterplatten ausgesät. Gentechnisch veränderte Zellen wurden durch Wachstum in Dulbeccos Modified Eagles Medium und 10 % foetalem Kälberserum und 150 - 500 ug/ml Zeocin während 3 bis 4 Wochen selektioniert Resistente Einzelkione wurden wie unten beschrieben analysiert.

### Fura-2-Messungen

Die Veränderungen der intrazellulären Calcium-Konzentration wurden mit Fura-2 gemessen. Eine Stammlösung mit 2 mM Fura-2-acetoxymethylester (Sigma) in Dimethylsulfoxid (DMSO) wurde auf eine Endkonzentration von 5 - 10 µM in serumfreiem Minimal Essentiellem Medium (MEM, Gibco) mit 1% Rinderserumalbumin and 5 mM Calciumchlorid verdünnt. Die Zellen wurden in einer Mikrotiterplane in dieser Lösung 45 bis 60 min lang inkubiert. Anschließend wurden die Zellen zweimal in N-(2-Hydroxyethyl)piperazine-N'-(2-ethanesulfonic acid) (5 mM HEPES) gepufferter Tyrode-Lösung (HEPES-gepufferte Salzlösung mit 130 mM NaCl, 5 mM KCl, 2 mM CaCl2, 1 mM MgCl2, 5 mM NaHCO3, 10 mM Glucose) gewaschen. 100 µl Tyrodepuffer wurde in die Vertiefungen der Mikrotiterplatte gegeben und die Zellen wurden unter einem Fluoreszenzmikroskop (Nikon Diaphot) abwechselnd mit Licht der Wellenlänge 340 nm und 380 nm bestrahlt. Eine Serie von Videobildern (Belichtungszeit pro Bild 100 ms) wurde mit Pausen von 3 Sekunden aufgenommen und als digitalisierte Bilder in einem Bildananlyse-Computer gespeichert (Leica, Quantimet 570). Nach Aufnahme von 8 Bildern (Meßpunkt 4.0 in Fig. 1) wurde Nikotin auf eine Endkonzentration von 500 µM zugegeben, und die Meßreihe fortgesetzt. Die Fluoreszenzintensität der Zellen bei Bestrahlung mit Licht der Wellenlänge 380 nm wurde durch die entsprechende Intensität bei 340 nm geteilt und so ein Verhältnis gebildet, das den relativen Anstieg der Calcium-Konzentration darstellt (Grynkiewicz et al. 1985).

### Literatur:

Bossy et al. (1988) Conservation of neural nicotinic acetylcholine receptors from Drosophila to vertebrate central nervous systems, EMBO J. 7, 611-618
Breer et al. (1987) Molecular properties and functions of insect acetylcholine receptors, J. Insect Physiol. 33, 771-790
Buckingham et al. (1997) Imidacloprid actions on insect neuronal acetylcholine receptors, J.Exp. Biol. 200, 2685-2692
Changeux et al. (1992) The functional architecture of the nicotinic acetylcholine receptor explored by affinity labelling and site-directed mutagenesis, Quarterly Review of Biophysics 25, 395-432
Claudio et al. (1983) Nucleotide and deduced amino acid sequences of Torpedo californica acetylcholine receptor g subunit, Proc. Natl. Acad. Sci. USA 80, 1111-1115
Devereux et al. (1984), Nucleic Acids Researeh 12, 387
Devillers-Thiery et al. (1983) Complete mRNA coding sequence of the acetylcholine binding α-subunit of Torpedo marmorata acetylcholine receptor: a model for the transmembrane organization of the polypeptide chain, Proc. Natl. Acad. Sci. USA 80, 2067-2071
Elgoyhen et al. (1997) US Pat. No. 5,683,912
Eastham et al. (1998) Characterisation of a nicotinic acetylcholine receptor from the insect Manduca sexta, Eur. J. Neurosci 10, 879-889
Grynkiewicz et al. (1985) A new generation of Ca2+ indicators with greatly improved fluorescence properties, J Biol Chem. 260, 3440-3450
Hay et al. (1997), P element insertion-dependent gene activation in the Drosophila eye, Proceedings of The National Academy of Sciences of The United States of America 94 (10), 5195-5200
Hermans-Borgmeyer et al. (1986) Primary structure of a developmentally regulated nicotinic acetylcholine receptor protein from Drosophila EMBO J. 5, 1503-1508
Heinemann et al. (1997) US Pat. No 5,591,590
Jespersen et al. (1997) Efficient Non-PCR-Mediated Overlap Extension of PCR Fragments by Exonuclease "End Polishing", Biotechniques, 23, 48-52
Lindstrom et al. (1997) US Pat. No. 5,599,709
Marshall et al. (1990) Sequence and functional expression of a single a subunit of an insect nicotinic acetylcholine receptor, EMBO J. 9, 4391-4398
Noda et al. (1982), Primary structure of α-subunit precursor of Torpedo californica acetylcholine receptor deduced from cDNA sequence, Nature 299, 793-797
Noda et al. (1983a), Primary structures of β- and δ-subunit precursor of Torpedo califomica acetylcholine receptor deduced from cDNA sequences, Nature 301, 251-255
Noda et al. (1983b), Structural homology of Torpedo californica acetylcholine receptor subunits, Nature 302, 528-532
Ortells et al. (1995), Evolutionary history of the ligand-gated ion-channel superfamily of receptors, Trends in Neurosience 18, 121-127
Plasterk (1996), The Tc1/mariner transposon family, Transposable Elements/Current Topics in Microbiology and Immunology 204, 125-143
Sambrook et al. (1989), Molecular Cloning, A Laboratory Manual, 2nd ed. Cold Spring Harbour Press
Sawruk et al. (1990a), EMBO J. 9, 2671-2677 Heterogeneity of Drosophila nicotinic acetylcholine receptors: SAD, a novel developmentally regulated α-subunit
Sawruk et al. (1990b), SBD, a novel structural subunit of the Drosophila nicotinic acetylcholine receptor, shares its genomic localization with two a-subunits, FEBS Lett. 273, 177-181
Schloß et al. (1988), Neuronal acetylcholine receptors of Drosophila: the ARD protein is a component of a high-affinity α-bungarotoxin binding complex, EMBO J 7, 2889-2984
Stetzer et al. (1996) Stable expression in HEK-293 cells of the rat α3/β4 subtype of neuronal nicotinic acetylcholine receptor, FEBS Lett. 397, 39-44
Zong et al. (1995) On the regulation of the expressed L-type calcium channel by cAMP-dependent phosphorylation, Pflügers Arch. - Eur. J. Physiol. 430, 340-347.

## Patentansprüche

1. Nukleinsäure umfassend eine Sequenz ausgewählt aus
(a) den Sequenzen gemäß SEQ ID NO: 1, SEQ ID NO: 3 oder SEQ ID NO: 5,
(b) zumindest 14 Basenpaare langen Teilsequenzen der unter (a) definierten Sequenzen,
(c) Sequenzen, welche an die unter (a) definierten Sequenzen hybridisieren in 2 x SSC bei 60°C, bevorzugt in 0,5 x SSC bei 60°C, besonders bevorzugt in 0,2 x SSC bei 60°C,
(d) Sequenzen, welche eine zumindest 70%ige Identität zu den unter (a) definierten Sequenzen zwischen Position 1295 und Position 2195 aus SEQ ID NO: 1 oder zwischen Position 432 und Position 1318 aus SEQ ID NO: 3 oder zwischen Position 154 und Position 1123 aus SEQ ID NO: 5 aufweisen,
(e) Sequenzen, welche zu den unter (a) definierten Sequenzen komplementär sind und
(f) Sequenzen, welche aufgrund der Degeneration des genetischen Codes für dieselbe Aminosäuresequenz kodieren wie die unter (a) bis (d) definierten Sequenzen.

2. Vektor umfassend zumindest eine Nukleinsäure gemäß Anspruch 1.

3. Vektor nach Anspruch 2, dadurch gekennzeichnet, daß je besagte Nukleinsäure funktionell mit regulatorischen Sequenzen verknüpft ist, die die Expression der Nukleinsäure in pro- oder eukaryotischen Zellen gewährleisten.

4. Wirtszelle enthaltend eine Nukleinsäure gemäß Anspruch 1 oder einen Vektor gemäß Anspruch 2 oder 3.

5. Wirtszelle nach Anspruch 4, dadurch gekennzeichnet, daß es sich um eine pro- oder eukaryotische Zelle handelt.

6. Wirtszelle nach Anspruch 5, dadurch gekennzeichnet, daß die prokaryotische Zelle E.coli ist.

7. Wirtszelle nach Anspruch 5, dadurch gekennzeichnet, daß die eukaryotische Zelle eine Säuger- oder Insektenzelle ist.

8. Polypeptid, welches von einer Nukleinsäure gemäß Anspruch 1 kodiert wird.

9. Acetylcholinrezeptor umfassend zumindest ein Polypeptid gemäß Anspruch 8.

10. Verfahren zur Herstellung eines Polypeptids gemäß Anspruch 8 umfassend
(a) das Kultivieren einer Wirtszelle gemäß einem der Ansprüche 4 bis 7 unter Bedingungen, die die Expression der Nukleinsäure gemäß Anspruch 1 gewährleisten, und
(b) die Gewinnung des Polypeptids aus der Zelle oder dem Kulturmedium.

11. Antikörper, welcher spezifisch mit dem Polypeptid gemäß Anspruch 8 oder dem Rezeptor gemäß Anspruch 9 reagiert.

12. Transgener Invertebrat enthaltend eine Nukleinsäure gemäß Anspruch 1.

13. Transgener Invertebrat nach Anspruch 12, dadurch gekennzeichnet, daß es sich um Drosophila melanogaster oder Caenorhabditis elegans handelt.

14. Verfahren zur Herstellung eines transgenen Invertebraten gemäß Anspruch 12 oder 13 umfassend das Einbringen einer Nukleinsäure gemäß Anspruch 1 oder eines Vektors gemäß Anspruch 2 oder 3.

15. Transgene Nachkommen eines Invertebraten gemäß Anspruch 12 oder 13.

16. Verfahren zur Herstellung einer Nukleinsäure gemäß Anspruch 1 umfassend die folgenden Schritte:
(a) Vollständige chemische Synthese aufan sich bekannte Weise oder
(b) chemische Synthese von Oligonukleotiden, Markieren der Oligonukleotide, Hybridisieren der Oligonukleotide an DNA einer InsektencDNA-Bank, Selektieren von positiven Klonen und Isolieren der hybridisierenden DNA aus positiven Klonen oder
(c) chemische Synthese von Oligonukleotiden und Amplifizierung der Ziel-DNA mittels PCR.

17. Regulatorische Region, welche natürlicherweise die Transkription einer Nukleinsäure gemäß Anspruch 1 in Insektenzellen kontrolliert und eine spezifische Expression gewährleistet.

18. Verfahren zum Auffinden neuer Wirkstoffe für den Pflanzenschutz, insbesondere von Verbindungen, welche die Leitungseigenschaften von Rezeptoren gemäß Anspruch 9 verändern, umfassend die folgenden Schritte:
(a) Bereitstellen einer Wirtszelle gemäß einem der Ansprüche 4 bis 7,
(b) Kultivieren der Wirtszelle in der Gegenwart einer Verbindung oder einer Probe, welche eine Vielzahl von Verbindungen umfaßt, und
(c) Detektieren veränderter Rezeptoreigenschaften.

19. Verfahren zum Auffinden einer Verbindung, die an Rezeptoren gemäß Anspruch 9 bindet, umfassend die folgenden Schritte:
(a) Inkontaktbringen einer Wirtszelle gemäß einem der Ansprüche 4 bis 7, eines Polypeptids gemäß Anspruch 8 oder eines Rezeptors gemäß Anspruch 9 mit einer Verbindung oder einem Gemisch von Verbindungen unter Bedingungen, die die Interaktion der Verbindung(en) mit der Wirtszelle, dem Polypeptid oder dem Rezeptor erlauben, und
(b) Bestimmen der Verbindung(en), die spezifisch an die Rezeptoren binden.

20. Verfahren zum Auffinden von Verbindungen, die die Expression von Rezeptoren gemäß Anspruch 9 verändern, umfassend die folgenden Schritte:
(a) Inkontaktbringen einer Wirtszelle gemäß einem der Ansprüche 4 bis 7 oder eines transgenen Invertebraten gemäß Anspruch 11 oder 12 mit einer Verbindung oder einem Gemisch von Verbindungen,
(b) Bestimmen der Rezeptorkonzentration, und
(c) Bestimmen der Verbindung(en), die die Expression des Rezeptors spezifisch beeinflussen.

21. Verwendung zumindest einer Nukleinsäure gemäß Anspruch 1, eines Vektors gemäß Anspruch 2 oder 3, einer regulatorischen Region gemäß Anspruch 16 oder eines Antikörpers gemäß Anspruch 11 zum Auffinden neuer Wirkstoffe für den Pflanzenschutz oder zum Auffinden von Genen, die für Polypeptide kodieren, welche am Aufbau von fünktionell ähnlichen Acetylcholinrezeptoren in Insekten beteiligt sind.
